# EUROPÄISCHE PATENTSCHRIFT

(11) **EP 0 785 187 B1**
(45) Veröffentlichungstag und Bekanntmachung des Hinweises auf die Patenterteilung: **20.06.2001**
(21) Anmeldenummer: 97100224.1
(22) Anmeldetag: 09.01.1997
(51) Int. Cl.: C07C 335/34, C07C 319/02, C07C 323/52, C07D 339/04, C07C 335/32, A61K 31/385

(54) **Herstellung von Salzen der 6,8-Bis(amidiniumthio)-octansäure**
Preparation of salts of 6,8-bis(amidiniumthio)octanoic acid
Préparation de sels d'acide 6,8-bis(amidiniumthio)octanoique

(30) Priorität: 19.01.1996 DE 19601787
(43) Veröffentlichungstag der Anmeldung: 23.07.1997
(73) Patentinhaber: ASTA Medica Aktiengesellschaft, 01277 Dresden (DE)
(72) Erfinder: Beisswenger, Thomas, Dr., 01445 Radebeul (DE); Gewald, Rainer, Dr., 01217 Dresden (DE); Olbrich, Alfred Dr., 33790 Halle/Westfalen (DE); Bethge, Horst, Dr., 63517 Rodenbach (DE); Hübner, Frank, Dr., 64372 Ober-Ramstadt (DE); Huthmacher, Klaus, Dr., 63571 Gelnhausen (DE); Klenk, Herbert, Dr., 63457 Hanau (DE); Möller, Roland, 63546 Hammersbach (DE); Rautenberg, Stephan, Dr., 63457 Hanau (DE); Sator, Gerhard, Dr., 64807 Dieburg (DE)
(74) Vertreter: Wibbelmann, Jobst, Dr., Dipl.-Chem.

(56) Entgegenhaltungen:
- EP-A- 0 427 247
- EP-A- 0 487 986
- EP-A- 0 763 533
- FR-A- 1 273 856
- GB-A- 996 703
- YADAV J S ET AL: "SYNTHESIS OF ALPHA-LIPOIC ACID-A HIGHLY USEFUL BIOLOGICALLY ACTIVE COMPOUND" JOURNAL OF SCIENTIFIC & INDUSTRIAL RESEARCH, Bd. 49, Nr. 8, August 1990, Seiten 400-409, XP000645882

## Beschreibung

Die Erfindung betrifft die Herstellung von Salzen der 6,8-Bis(amidiniumthio)octansäure, deren Enantiomeren und Estern dieser Verbindungen. 6,8-Bis(amidiniumthio)octansäure kann durch Hydrolyse der Isothiuronium-Gruppierung zur 6,8-Dimercaptooctansäure umgesetzt werden (auch als Dihydroliponsäure bezeichnet) die ihrerseits als Vorstufe für die pharmakologisch wirksame α-Liponsäure dient.

Das R-Enantiomer der α-Liponsäure ist ein Naturstoff, der in praktisch allen tierischen und pflanzlichen Zellen vorkommt. Als Coenzym bei der oxidativen Decarboxylierung von α-Ketocarbonsäuren (z.B. Brenztraubensäure) ist α-Liponsäure von essentieller physiologischer Bedeutung. Eine wichtige pharmakologische Indikation der razemischen α-Liponsäure ist die diabetische Polyneuropathie. Bei den reinen optischen Isomeren der α-Liponsäure (R- und S-Form, d.h. R-α-Liponsäure und S-α-Liponsäure) ist im Gegensatz zu dem Razemat das R-Enantiomer vorwiegend antiphlogistisch und das S-Enantiomer vorwiegend antinociceptiv wirksam (EP 0 427 247, 08.11.90). Neben der Synthese von razemischer α-Liponsäure ist daher auch die Synthese der reinen Enantiomere zum gezielten pharmazeutischen Einsatz von großer Wichtigkeit.

Die bisher bekannten Syntheseverfahren nutzen verschiedene Varianten zur gezielten Einführung von Mercaptogruppen an Derivaten oder Vorläufern von Octansäure, die in mehreren Literaturstellen referiert werden. (Übersichtsartikel: J.S. Yadav et al., J. Sci. Ind. Res. 1990, 49, 400; sowie: A.G. Tolstikov et al., Bioorg. Khim. 1990, 16, 1670; L. Dasaradhi et al., J. Chem. Soc., Chem. Commun. 1990, 729; A.S. Gopalan et al., J. Chem. Perkin Trans. 1 1990, 1897; A.S. Gopalan et al., Tetrahedron Lett. 1989, 5705; EP 0487986 A2, 14.11.91; E. Walton et al. J. Am. Chem. Soc. 1955, 77, 1955; D.S. Acker und W.J. Wayne, J. Am. Chem. Soc. 1957, 79, 6483; L.G. Chebotareva und A.M. Yurkevich, Khim.-Farm. Zh. 1980, 14, 92).

Problematisch bei allen beschriebenen Herstellungsvarianten sind einerseits vielstufige Synthesesequenzen mit teilweise niedrigen Reaktionsausbeuten, wie z.B. das Verfahren nach der FR-A-1 273 856, die eine wirtschaftliche Arbeitsweise nicht erlauben, oder die Instabilität von Zwischenstufen, die ihre technische Reinigung verhindert. Die benötigte Reinheit für pharmazeutische Ware läßt sich daher erst durch aufwendige Endreinigung durch Destillation der Dihydroliponsäure (6,8-Dimercaptooctansäure) **II** bzw. durch mehrfache Kristallisation der empfindlichen α-Liponsäure **I**, die zu erheblichen Produktverlusten führt, erreichen. Beispielhaft läßt sich die GB 996,703 mit der dort beschriebenen Umsetzung von 6-Hydroxy-Δ7-octensäure oder ihrer Ester anführen, wobei durch Destillation Ausbeuten von 23 bis 51% d.Th. an Dimercaptooctansäure **II** bzw. α-Liponsäure **I** erzielt wurden.

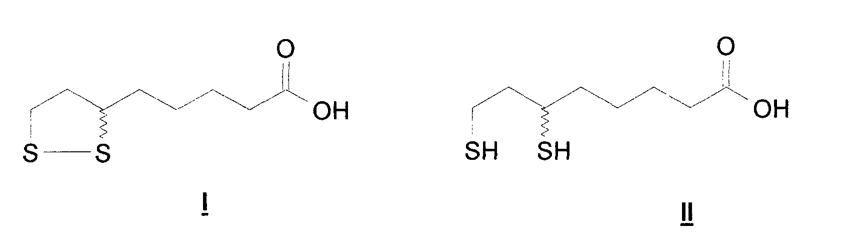

Demnach liegt der Erfindung die Aufgabe zugrunde, Zwischenprodukte für die Herstellung von α-Liponsäure zu schaffen, die sich leicht reinigen lassen und deren Verwendung zu hohen Ausbeuten des Endprodukts α-Liponsäure führt.

Diese Aufgabe wird erfindungsgemäß durch ein Verfahren zur Herstellung von Verbindungen der allgemeinen Formel **III** 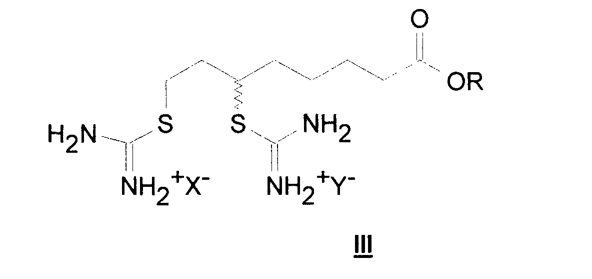 in der R ein Wasserstoffatom oder eine Alkylgruppe mit 1 bis 3 Kohlenstoffatomen bedeutet sowie X⁻ und Y⁻ gleich oder verschieden sind und ein Anion einer starken Säure, vorzugsweise einer Mineralsäure, Alkyl- oder Arylsulfonsäure oder Carbonsäure bedeuten, und deren Enantiomeren, bei dem enantiomerenreine oder razemische 8-Chlor-6-sulfonyloxyoctansäure oder deren C₁-C₃-Alkylester mit Thioharnstoff umgesetzt werden, gelöst.

Beispiele für besonders geeignete Säuren sind Chlorwasserstoffsäure sowie Methylsulfonsäure, Naphthalin-1,5-disulfonsäure und Terephthalsäure.

Bevorzugte Sulfonyloxy-Substituenten sind Methylsulfonyloxy-, 4-Methylphenylsulfonyloxy- und Perfluoralkylsulfonyloxy-Gruppen.

Aus dem entstandenen Reaktionsgemisch können die Verbindungen der Formel **III** aus wäßriger Lösung isoliert werden. Durch Zusatz von Sulfonsäuren, Mineralsäuren oder Carbonsäuren lassen sich die in kristalliner oder öliger Form vorliegenden Salze erhalten.

Mit dem erfindungsgemäßen Verfahren gelingt es überraschenderweise, aus Verbindungen der allgemeinen Formel **III** durch schonende Umsetzung razemische oder enantiomerenreine Dimercaptooctansäure **II** herzustellen, die dann aufgrund der erzielten Reinheit leicht und in guten Ausbeuten zu razemischer oder enantiomerenreiner α-Liponsäure **I** oxidiert werden kann.

Beim erfindungsgemäßen Verfahren wird beispielsweise so vorgegangen, daß razemische 8-Chlor-6-hydroxyoctansäure oder ihre Ester entsprechend der beschriebenen Methode von Thursin und Fiymonoto (GB 933.809) mit Sulfonsäurechloriden zur razemischen 8-Chlor-6-sulfonyloxyoctansäure oder deren Estern umgesetzt werden. Analoge Umsetzungen können auch mit 6,8-Dihydroxyoctansäure und deren Estern zu 6,8-Bis(sulfonyloxy)octansäure und deren Estern durchgeführt werden [Rama Rao et al., Synth. Commun., 17 (1987) 1339]. Diese Verbindungen können dann in Lösung mit Thioharnstoff direkt zu den Salzen der 6,8-Bis(amidiniumthio)octansäure oder ihrer Ester **III** reagieren. Man kann jedoch auch über die enantiomerenreinen (+)-8-Chlor-6-sulfonyloxyoctansäure bzw. (-)-8-Chlor-6-sulfonyloxyoctansäure oder deren Ester in guten Ausbeuten stereospezifisch unter vollständiger Inversion am chiralen Zentrum zu den Salzen von optisch aktiven (-)-6,8-Bis(amidiniumthio)octansäure bzw. (+)-6,8-Bis(amidiniumthio)octansäure oder deren Estern gelangen. Die Herstellung der Salze der optisch aktiven 6,8-Bis(amidiniumthio)octansäure erfolgt hierbei in einem analogen Verfahren, wie dies für die razemischen Gemische beschrieben wird.

Als Lösemittel für diese Reaktionen kommen organische Lösemittel sowie Gemische derselben untereinander oder mit Wasser in Frage. Beispiele für geeignete organische Lösemittel sind: Alkylalkohole, vorzugsweise mit Kettenlängen von 1 bis 6, Alkylcarbonsäuren, vorzugsweise mit Kettenlängen von 2 bis 3 und Ameisensäure, oder die Ester aus den genannten Alkoholen und Carbonsäuren mit Kettenlängen von 2 bis 3, gesättigte und ungesättigte lineare und zyklische Kohlenwasserstoffe, vorzugsweise Alkylbenzole wie Toluol oder Xylole, oder Alkane wie beispielsweise Pentan oder Cyclohexan, oder halogenierte Kohlenwasserstoffe.

Die erhaltenen Salze von 6,8-Bis(amidiniumthio)octansäure oder deren Estern **III** können weiterhin analog zur an sich literaturbekannten Art und Weise durch Alkalihydroxide, Erdalkalihydroxide oder tert. Aminbase zur razemischen oder enantiomerenreinen 6,8-Dimercaptooctansäure **II** umgesetzt werden (Organikum 12. Auflage (1973), S.223, VEB Deutscher Verlag der Wissenschaften, Berlin.) Dabei lassen sich aus den optisch reinen Salzen **III** anschließend (-)-6,8-Dimercaptooctansäure **II** bzw. (+)-6,8-Dimercaptooctansäure **II** oder R-α-Liponsäure **I** bzw. S-α-Liponsäure **I** in vorzüglicher optischer Reinheit (e.e.> 99%, chirale HPLC) als pharmazeutisch einsetzbare Verbindungen erhalten.

Die vorliegende Erfindung ermöglicht es, die Salze der Razemate und der Enantiomere der 6,8-Bis(amidiniumthio)octansäure sowie der Ester dieser Verbindungen auf einfache und wirtschaftliche Weise in hoher Reinheit zugänglich zu machen. Dies wird durch nachfolgende Beispiele näher erläutert.

Die Reinheit der optischen Isomere wurde mittels der spezifischen optischen Drehwerte bestimmt. Die relativen Gehalte der optischen Isomere der 8-Chlor-6-hydroxyoctansäure und der α-Liponsäure **IIa/b** wurden außerdem durch HPLC an optisch aktiven Säulen untersucht und mit einer Nachweisgrenze von >0,5% bestimmt.

### Beispiel 1

In 50 ml Ethanol und 85 ml Toluol, wurden 28,7 g (0,10 mol) 8-Chlor-6-methansulfonyloxyoctansäuremethylester [286,453] aufgelöst und 15,2 g (0,20 mol) Thioharnstoff zugegeben. Die Mischung wurde während 4 h zum Rückfluß erhitzt und dann bis zu einer Kopftemperatur von 110 °C andestilliert. Zum Reaktionsgemisch wurden 130 ml Wasser zugegeben, abgekühlt und die Phasen getrennt. Die wässrige Phase enthielt das 6,8-Bis(amidiniumthio)octansäuremethylester chloridmethylsulfonat, das mit 2,0 ml konzentrierter Salzsäure versetzt wurde und 2 h bis zu einer Kopftemperatur von 100 °C andestilliert wurde. Die Lösung wird eingeengt und zuletzt im Hochvakuum getrocknet. Das zürückbleibende Öl enthält das 6,8-Bis(amidiniumthio)octansäure chloridmethylsulfonat.

Ausbeute: 33,0 g (78 % d.Th.) [424,98] 6,8-Bis(amidiniumthio)octansäure chlorid methylsulfonat

| C₁₁H₂₅N₄O₅S₃Cl | | | | | | | |
|---|---|---|---|---|---|---|---|
| Elementaranalyse: | ber.: | C 31,09 | H 5,93 | N 13,18 | S 22,63 | Cl 8,3 | O 18,82 |
| | gef.: | C 29,69 | H 6,15 | N 12,16 | S 21,86 | Cl 8,5; | 2,5 % H₂O |

IR: 1710 s, 1660 s, 1550 m, 1430 s, 1240 m, 1200/1170 s, 1050 s, 790 s.

¹H-NMR d₆-DMSO: 12,0 s (1H) COOH; 9,25 s (8H) NH₂; 3,97 m (1H) CH-S; 3,32 m (2H) CH₂-S; 2,46 s (3H) SO₃CH₃; 2,25 m (2H) SCH₂CH₂; 2,00 m (2H) CH₂; 1,78/1,58 m (2H) CH₂; 1,53 m (2H) CH₂; 1,40 m (2H) CH₂; ¹³C-NMR d₆-DMSO: 174,25 COOH; 169,70/169,19 S-C(NH₂)₂; 45,32 S-CH; 39,83 CH₃SO₃; 33,72; 33,45; 32,84; 27,07; 25,32; 24,14.

6,8-Bis(amidiniumthio)octansäuremethylester chloridmethylsulfonat

¹H-NMR d₆-DMSO: 3,92 m (1H) CH-S; 3,57s(3H) OCH₃; 3,27 m (2H) CH₂-S; 2,45 s (3H) SO₃CH₃; 2,4 m (2H) SCH₂CH₂; 1,97 m (2H) CH₂; 1,76/1,52 m (2H) CH₂; 1,50 m (2H) CH₂; 1,35 m (2H) CH₂;

### Beispiel 2:

In 50 ml Ethanol, 85 ml Toluol und 10 ml Wasser wurden 28,7 g (0,10 mol) 8-Chlor-6-methansulfonyloxyoctansäuremethylester aufgelöst und 15,2 g (0,20 mol) Thioharnstoff zugegeben. Die Mischung wurde während 4 h zum Rückfluß erhitzt und dann bis zu einer Kopftemperatur von 110 °C andestilliert. Zum Reaktionsgemisch wurden 130 ml Wasser zugegeben, abgekühlt und die Phasen getrennt. Die Lösung wurde eingeengt und zuletzt im Hochvakuum getrocknet. Das zürückbleibende Öl enthielt das 6,8-Bis(amidiniumthio)octansäure chloridmethylsulfonat.

Ausbeute: 34,0 g (80 % d.Th.) [424,98] 6,8-Bis(amidiniumthio)octansäure chlorid methylsulfonat.

### Beispiel 3:

In 50 ml Ethanol, 85 ml Toluol und 10 ml Wasser wurden 28,7 g (0,10 mol) 8-Chlor-6-methansulfonyloxyoctansäuremethylester, in 10 ml Toluol gelöst, aufgelöst und 15,2 g (0,20 mol) Thioharnstoff zugegeben. Die Mischung wurde 4 Std. zum Rückfluß erhitzt und anschließend bis zu einer Kopftemperatur von 110 °C andestilliert. Zur Reaktionsmischung wurden 130 ml Wasser zugegeben, abgekühlt und die Phasen getrennt. Die wässrige Phase wurde mit einer Lösung von 33,2 g (0,10 mol) 1,5-Naphthalindisulfonsäure Dinatriumsalz, in 100 ml Waser gelöst, versetzt. Die entstehende Suspension wurde bei Raumtemperatur über Nacht gerührt und das Salz abfiltriert. Das Salz wurde anschließend bei 70 °C im Vakuum getrocknet. Man erhielt das 6,8-Bis(amidiniumthio)octansäure naphthalin-1,5-disulfonat.

Ausbeute: 49,4 g (85 % d.Th.) 6,8-Bis(amidiniumthio)octansäure naphthalin-1,5-disulfonat [580,70].

| C₂₀H₂₈N₄O₈S₄ | | | | | | |
|---|---|---|---|---|---|---|
| Elementaranalyse: | ber.: | C 41,37 | H 4,86 | N 9,65 | S 22,08 | O 22,04 |
| | gef.: | C 41,36 | H 4,80 | N 9,58 | S 22,17 | |

IR: 3170 s, 1730 s, 1660 s, 1440 m, 1410 s, 1210 m, 1180/1150 s, 1030 s, 810 s, 770 s.

¹H-NMR d₆-DMSO: 12,0 s (1H) COOH; 9,02 (8H) NH₂; 8,88 d (2H) CH=; 8,00 d (2H) CH=; 7,45 t (2H) CH=; 3,80 m (1H) CH-S; 3,20 m (2H) CH₂-S; 2,22 m (2H) SCH₂CH₂; 1,95 m (2H) CH₂; 1,72/1,55 m (2H) CH₂; 1,50 m (2H) CH₂; 1,34 m (2H) CH₂;

### Beispiel 4:

In 50 ml Ethanol, 85 ml Toluol und 10 ml Wasser wurden 28,7 g (0,10 mol) 8-Chlor-6-methansulfonyloxyoctansäuremethylester aufgelöst und 15,2 g (0,20 mol) Thioharnstoff zugegeben. Die Mischung wurde während 4 h zum Rückfluß erhitzt und dann bis zu einer Kopftemperatur von 110 °C andestilliert. Zum Reaktionsgemisch wurden 130 ml Wasser zugegeben, abgekühlt und die Phasen getrennt. Die wässrige Phase wurde mit einer Lösung von 21 g (0,10 mol) Terephthalsäure Dinatriumsalz, in 100 ml Waser gelöst, versetzt. Die entstehende Suspension wurde bei Raumtemperatur 4 h gerührt und das Salz abfiltriert. Das Salz wurde anschließend bei 70 °C im Vakuum getrocknet. Man erhielt das 6,8-Bis(amidiniumthio)octansäure terephthalat.

Ausbeute: 49,4 g (85 % d.Th.) 6,8-Bis(amidiniumthio)octansäure terephthalat [458,55].

| C₁₈H₂₆N₄O₆S₂ | | | | | | |
|---|---|---|---|---|---|---|
| Elementaranalyse: | ber.: | C 47,15 | H 5,72 | N 12,22 | S 13,98 | O 20,93 |
| | gef.: | C 47,53 | H 5,78 | N 11,13 | S 13,03 | |

IR (KBr): 3250 s, 1690 s, 1580 s, 1430 m, 1410 s, 1370 m, 1290 s.

¹H-NMR D₂O: 7,70 (4H) Ph-H; 4,55 COOH/H₂O/NH₂; 3,50 m (1H) CH-S; 3,05 m (2H) CH₂-S; 2,02 m (2H) SCH₂CH₂; 1,95/1,90 m (2H) CH₂; 1,60/1,55 m (2H) CH₂; 1,37 m (2H) CH₂; 1,25 m (2H) CH₂;

### Beispiel 5:

In 50 ml Ethanol, 85 ml Toluol und 10 ml Wasser wurden 36,2 g (0,10 mol) 8-Chlor-6-(4-methylphenylsulfonyloxy)octansäuremethylester [362,453] aufgelöst und 15,2 g (0,20 mol) Thioharnstoff zugegeben. Die Mischung wurde während 4 h zum Rückfluß erhitzt und dann bis zu einer Kopftemperatur von 110 °C andestilliert. Zum Reaktionsgemisch wurden 130 ml Wasser zugegeben, abgekühlt und die Phasen getrennt. Die wässrige Phase, die das 6,8-Bis(amidiniumthio)octansäure chlorid 4-methylphenylsulfonat enthielt, wurde mit einer Lösung von 33,2 g (0,10 mol) 1,5-Naphthalindisulfonsäure Dinatriumsalz, in 100 ml Waser gelöst, versetzt. Die entstehende Suspension wurde bei Raumtemperatur 4 h gerührt und das Salz abfiltriert. Das Salz wurde anschließend bei 70 °C im Vakuum getrocknet. Man erhielt das 6,8-Bis(amidiniumthio)octansäure naphthalin-1,5-disulfonat.

Ausbeute: 52,8 g (91 % d.Th.) 6,8-Bis(amidiniumthio)octansäure naphthalin-1,5-disulfonat [580,70]

### Beispiel 6

In 50 ml Ethanol, 85 ml Toluol und 10 ml Wasser wurden 34,6 g (0,10 mol) 6,8-bis(methylsulfonyloxy)octansäuremethylester [346,4] aufgelöst und 15,2 g (0,20 mol) Thioharnstoff zugegeben. Die Mischung wurde bis zu einer Kopftemperatur von 110 °C andestilliert und dann während 4 h zum Rückfluß erhitzt. Zum Reaktionsgemisch wurden 130 ml Wasser zugegeben, abgekühlt und die Phasen getrennt. Die wässrige Phase, die das 6,8-Bis(amidiniumthio)octansäure dimesylat enthielt, wurde mit einer Lösung von 33,2 g (0,10 mol) 1,5-Naphthalindisulfonsäure Dinatriumsalz, in 100 ml Waser gelöst, versetzt. Die entstehende Suspension wurde bei Raumtemperatur 4 h gerührt und das Salz abfiltriert. Das Salz wurde anschließend bei 70 °C im Vakuum getrocknet. Man erhielt das 6,8-Bis(amidiniumthio)octansäure naphthalin-1,5-disulfonat.

Ausbeute: 52,8 g (91 % d.Th.) 6,8-Bis(amidiniumthio)octansäure naphthalin-1,5-disulfonat [580,70]

### Beispiel 7:

21,25 g (0,0366 mol) 6,8-Bis(amidiniumthio)octansäure naphthtalin-1,5-disulfonat wurden in 200 m! Ethanol und 200 ml Wasser aufgelöst und bei 70 °C mit 7,62 g (0,0366 mol) Bariumchlorid, in 100 ml Wasser gelöst, versetzt. Die entstehende Suspension wurde 4 Stunden bei 70 °C gerührt und der Feststoff abfiltriert. Die flüssige Phase wurde eingeengt und ergab einen salzhaltigen öligen Rückstand. Dieser wurde mit 50 ml Ethanol bei 60 °C ausgerührt und abfiltriert. Die ethanolische Lösung wurde zur Trockene eingeengt und im Hochvakuum von Lösemitteln befreit.

Ausbeute: 13,2 g (0,036 mol) 97 % d.Th. 6,8-Bis(amidiniumthio)octansäure dichlorid [365,34]

| C₁₀H₂₂N₄O₂S₂Cl₂ | | | | | | | |
|---|---|---|---|---|---|---|---|
| Elementaranalyse: | ber.: | C 32,88 | H 6,07 | N 15,34 | S 17,55 | Cl 19,4 | O 8,76 |
| | gef.: | C 32,49 | H 6,48 | N 14,92 | S 17,24 | Cl 19,19; | 1,13 % H₂O |

IR: 1710 s, 1640 s, 1530 m, 1430 s, 1240 m, 1200/1180 s, 1070 s.

¹H-NMR d₆-DMSO: 12,0 (1H) COOH; 9,3 s (8H) NH₂; 4,12 m (1H) CH-S; 3,40 m (2H) CH₂-S; 2,27 m (2H) SCH₂CH₂; 2,00 m (2H) CH₂; 1,80/1,56 m (2H) CH₂; 1,53 m (2H) CH₂; 1,43/1,36 m (2H) CH₂; ¹³C-NMR d₆-DMSO: 174,13 COOH; 169,69/169,22 S-C(NH₂)₂; 45,10 S-CH; 33,96; 33,35; 32,58; 26,92; 25,25; 24,02.

### Beispiel 8:

21,25 g (0,0366 mol) 6,8-Bis(amidiniumthio)octansäure naphthalin-1,5-disulfonat wurden in 200 ml Ethanol und 200 ml Wasser aufgelöst und bei 70 °C mit 7,62 g (0,0366 mol) Bariummethansulfonat, in 100 ml Wasser gelöst, versetzt. Die entstehende Suspension wurde 4 Stunden bei 70 °C gerührt und der Feststoff abfiltriert. Die flüssige Phase wurde eingeengt und ergab einen salzhaltigen öligen Rückstand. Dieser wurde mit 50 ml Ethanol bei 60 °C ausgerührt und abfiltriert. Die ethanolische Lösung wurde zur Trockene eingeengt und im Hochvakuum von Lösemitteln befreit.

Ausbeute: 13,2 g (0,036 mol) 98 % d.Th. 6,8-Bis(amidiniumthio)octansäure bis(methansulfonat) [484,62]

### Beispiel 9:

58,0 g (0,10 mol) 6,8-Bis(amidiniumthio)octansäure naphthalin-1,5-disulfonat [580,70] wurde in 100 ml Wasser suspendiert und 40 ml (0,50 mol) Natronlauge (50 %ig) innerhalb von 20 min zugetropft. Man erhielt eine Suspension, die 1 Std. auf 40 °C erwärmt wurde. Anschließend wurde mit 550 ml Wasser verdünnt. Die Lösung wurde mit 62,5 g (0,75 mol) konzentrierter Salzsäure angesäuert und 3 mal mit 100 ml Toluol extrahiert. Die vereinigten organischen Phasen wurden im Vakuum zur Trockene eingeengt.

Ausbeute: 20,2 g (97 % d.Th.) 6,8-Dimercaptooctansäure

### Beispiel 10:

20,0 g 6,8-Dimercaptooctansäure wurden in 100 ml wäßriger Natronlauge (0,1 mol) gelöst und mit Wasserstoffperoxid-Lösung oxidiert. Nach Zugabe von 200 ml Toluol wurde mit Salzsäure unter Rühren auf einen pH-Wert von 1 angesäuert und die organische Phase isoliert.

Ausbeute: Die organische Phase enthielt quantitativ die α-Liponsäure

### Beispiel 11:

In 50 ml Ethanol und 85 ml Toluol wurden 28,7 g (0,10 mol) (+)-8-Chlor-6-methansulfonyloxyoctansäuremethylester [∝]_{D}²⁰ = 32,9° (c = 1,0; Ethanol) [286,453], aufgelöst und 15,2 g (0,20 mol) Thioharnstoff zugegeben. Die Mischung wurde während 4 h zum Rückfluß erhitzt und dann bis zu einer Kopftemperatur von 110 °C andestilliert. Zum Reaktionsgemisch wurden 130 ml Wasser zugegeben, abgekühlt und die Phasen getrennt. Die wässrige Phase enthielt das (+)-6,8-Bis(amidiniumthio)octansäuremethylester chloridmethylsulfonat, das mit 2,0 ml konzentrierter Salzsäure versetzt wurde und 2 h bis zu einer Kopftemperatur von 100 °C andestilliert wurde. Die Lösung, die das (+)-6,8-Bis(amidiniumthio)octansäure chloridmethylsulfonat enthielt, wurde mit einer Lösung von 33,2 g (0,10 mol) 1,5-Naphthalindisulfonsäure Dinatriumsalz, in 100 ml Waser gelöst, versetzt. Die entstehende Suspension wurde bei Raumtemperatur 4 h gerührt und das Salz abfiltriert. Das Salz wurde anschließend bei 70 °C im Vakuum getrocknet. Man erhielt das (+)-6,8-Bis(amidiniumthio)octansäure naphthalin-1,5-disulfonat.

Ausbeute: 52,8 g (91 % d.Th.) (+)-6,8-Bis(amidiniumthio)octansäure naphthalin-1,5-disulfonat [580,70].

| C₂₀H₂₈N₄O₈S₄ | | | | | | |
|---|---|---|---|---|---|---|
| Elementaranalyse: | ber.: | C 41,37 | H 4,86 | N 9,65 | S 22,08 | O 22,04 |
| | gef.: | C 41,17 | H 4,77 | N 9,63 | S 22,13 | |

IR: 3170 s, 1730 s, 1660 s, 1440 m, 1410 s, 1210 m, 1180/1150 s, 1030 s, 810 s, 770 s.

¹H-NMR d₆-DMSO: 12,0 (1H) COOH; 9,2 (8H) NH₂; 8,90 d (2H) CH=; 7,97 d (2H) CH=; 7,43 t (2H) CH=; 3,78 m (1H) CH-S; 3,18 m (2H) CH₂-S; 2,21 m (2H) SCH₂CH₂; 1,94 m (2H) CH₂; 1,70/1,55 m (2H) CH₂; 1,51 m (2H) CH₂; 1,37 m (2H) CH₂;
[α]_{D}²⁰ = + 5,7 ° (c= 1,2; DMSO)

### Beispiel 12:

58,0 g (0,10 mol) (+)-6,8-Bis(amidiniumthio)octansäure naphthalin-1,5-disulfonat [580,70] wurde unter Stickstoffinertisierung in 100 ml Wasser suspendiert und 40 g (0,50 mol) Natronlauge (50 %ig) innerhalb von 20 min zugetropft. Man erhielt eine Suspension, die 1 Std. auf 40 °C erwärmt wurde. Anschließend wurde mit 550 ml Wasser verdünnt. Die Lösung wurde mit 62,5 g (0,75 mol) konzentrierter Salzsäure angesäuert und 3 mal mit 100 ml Toluol extrahiert. Die vereinigten organischen Phasen wurden im Vakuum zur Trockene eingeengt.

Ausbeute: 20,2 g (97 % d.Th.) (-)-6,8-Dimercaptooctansäure

Enantiomerenreinheit ee.: > 99%
[α]_{D}²⁰ = -13,5° (c = 1,0; Ethanol)

### Beispiel 13:

In 50 ml Ethanol und 85 ml Toluol wurden 28,7 g (0,10 mol) (+)-8-Chlor-6-methansulfonyloxyoctansäuremethylester, [∝]_{D}²⁰ = +32,8° (c = 1,0; Ethanol) [286,453], aufgelöst und 15,2 g (0,20 mol) Thioharnstoff zugegeben. Die Mischung wurde während 4 h zum Rückfluß erhitzt und dann bis zu einer Kopftemperatur von 110 °C andestilliert. Zum Reaktionsgemisch wurden 130 ml Wasser zugegeben, abgekühlt und die Phasen getrennt. Die wässrige Phase enthielt das (+)-6,8-Bis(amidiniumthio)octansäuremethylester chloridmethylsulfonat, das mit 2,0 ml konzentrierter Salzsäure versetzt wurde und 2 h bis zu einer Kopftemperatur von 100 °C andestilliert wurde. Die Lösung, die das (+)-6,8-Bis(amidiniumthio)octansäure chloridmethylsulfonat enthielt, wurde mit einer Lösung von 33,2 g (0,10 mol) 1,5-Naphthalindisulfonsäure Dinatriumsalz, in 100 ml Wasser gelöst, versetzt. Die entstehende Suspension wurde bei Raumtemperatur 4 h gerührt und das Salz abfiltriert. Das Salz wurde anschließend bei 70 °C im Vakuum getrocknet. Man erhielt das (+)-6,8-Bis(amidiniumthio)octansäure naphthalin-1,5-disulfonat.

Ausbeute: 52,8 g (91 % d.Th.) (+)-6,8-Bis(amidiniumthio)octansäure naphthalin-1,5-disulfonat [580,70].

| C₂₀H₂₈N₄O₈S₄ | | | | | | |
|---|---|---|---|---|---|---|
| Elementaranalyse: | ber.: | C 41,37 | H 4,86 | N 9,65 | S 22,08 | O 22,04 |
| | gef.: | C 41,13 | H 4,73 | N 9,59 | S 22,19 | |

IR: 3170 s, 1730 s, 1660 s, 1440 m, 1410 s, 1210 m, 1180/1150 s, 1030 s, 810 s, 770 s.

¹H-NMR d₆-DMSO: 12,0 s (1H) COOH; 9,2 s (8H) NH₂; 8,90 d (2H) CH=; 7,97 d (2H) CH=; 7,43 t (2H) CH=; 3,78 m (1H) CH-S; 3,18 m (2H) CH₂-S; 2,21 m (2H) SCH₂CH₂; 1,94 m (2H) CH₂; 1,70/1,55 m (2H) CH₂; 1,51 m (2H) CH₂; 1,37 m (2H) CH₂;
[α]_{D}²⁰ = +5,6 ° (c= 1,0; DMSO)

### Beispiel 14:

58,0 g (0,10 mol) (+)-6,8-Bis(amidiniumthio)octansäure naphthalin-1,5-disulfonat [580,70] wurde unter Stickstoffinertisierung in 100 ml Wasser suspendiert und 40 g (0,50 mol) Natronlauge (50 %ig) innerhalb von 20 min zugetropft. Man erhielt eine Suspension, die 1 Std. auf 40 °C erwärmt wurde. Anschließend wurde mit 550 ml Wasser verdünnt. Die Lösung wurde mit 62,5 g (0,75 mol) konzentrierter Salzsäure angesäuert und 3 mal mit 100 ml Toluol extrahiert. Die vereinigten organischen Phasen wurden im Vakuum zur Trockene eingeengt.

Ausbeute: 20,2 g (97 % d.Th.) (-)-6,8-Dimercaptooctansäure

Enantiomerenreinheit ee.: > 99%
[α]_{D}²⁰ = -13,7° (c = 1,5; Ethanol)

### Beispiel 15:

58,0 g (0,10 mol) (-)-6,8-Bis(amidiniumthio)octansäure naphthalin-1,5-disulfonat [580,70] wurde in 100 ml Wasser suspendiert und 40 g (0,50 mol) Natronlauge (50 %ig) innerhalb von 20 min zugetropft. Man erhielt eine Suspension, die 1 Std. auf 40 °C erwärmt wurde. Anschließend wurde mit 550 ml Wasser verdünnt. Die Lösung wurde mit 62,5 g (0,75 mol) konzentrierter Salzsäure angesäuert und 3 mal mit 100 ml Toluol extrahiert. Die Toluolextrakte wurden unter Rühren mit 100 ml wäßriger Natronlauge (0,1 mol) versetzt und mit Wasserstoffperoxid-Lösung oxidiert. Anschließend wurde mit Salzsäure unter Rühren auf einen pH-Wert von 1 angesäuert und die organische Phase isoliert. Die organische Phase wurde zur Trockene eingedampft und im Vakuum getrocknet.

Ausbeute: 19,2 g (93 % d.Th.) S-(-)-α-Liponsäure

Enantiomerenreinheit ee.: > 99%
[α]_{D}²⁰ = -119° (c = 1; Ethanol)

Smp.: 49-50 °C.

## Patentansprüche

1. Verfahren zur Herstellung einer Verbindung nach der allgemeinen Formel **III** 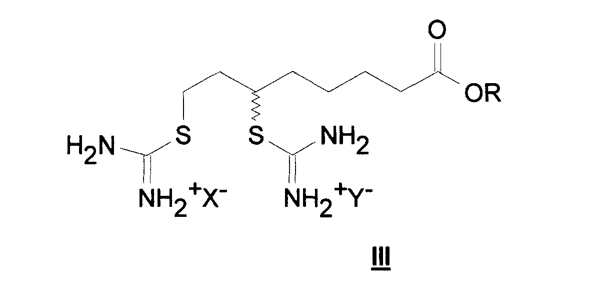 in der R ein Wasserstoffatom oder eine Alkylgruppe mit 1 bis 3 Kohlenstoffatomen bedeutet sowie X⁻ und Y⁻ gleich oder verschieden sind und ein Anion einer Mineralsäure, Alkyl- oder Arylsulfonsäure oder Carbonsäure bedeuten, und deren Enantiomeren, bei dem enantiomerenreine oder razemische 8-Chlor-6-sulfonyloxyoctansäure oder deren C₁-C₃-Alkylester mit Thioharnstoff umgesetzt werden.

2. Verfahren nach Anspruch 1, bei dem als Sulfonyloxy-Substituenten Methylsulfonyloxy-, 4-Methylphenylsulfonyloxy-, Perfluoralkylsulfonyloxy-Gruppen eingesetzt werden.

3. Verfahren nach Anspruch 1 oder 2, bei dem aus dem entstandenen Reaktionsgemisch die Verbindungen der Formel **III** nach Anspruch 1 in wäßriger Lösung als Salze von Sulfonsäuren, Mineralsäuren oder Carbonsäuren in kristalliner oder öliger Form isoliert werden.

## Claims

1. Process for the preparation of a compound according to the general formula **III** 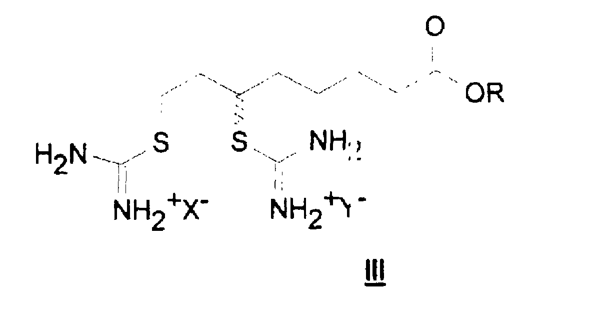 in which R is a hydrogen atom or an alkyl group having 1 to 3 carbon atoms, and X⁻ and Y⁻ are identical or different and are an anion of a mineral acid, alkyl- or arylsulphonic acid or carboxylic acid, and their enantiomers, in which enantiomerically pure or racemic 8-chloro-6-sulphonyloxyoctanoic acid or its C₁-C₃-alkyl esters are reacted with thiourea.

2. Process according to Claim 1, in which methylsulphonyloxy, 4-methylphenylsulphonyloxy and perfluoroalkylsulphonyloxy groups are employed as sulphonyloxy substituents.

3. Process according to Claim 1 or 2, in which the compounds of the formula **III** according to Claim 1 are isolated from the resulting reaction mixture in crystalline or oily form in aqueous solution as salts of sulphonic acids, mineral acids or carboxylic acids.

## Revendications

1. Procédé pour la préparation d'un composé de formule générale III 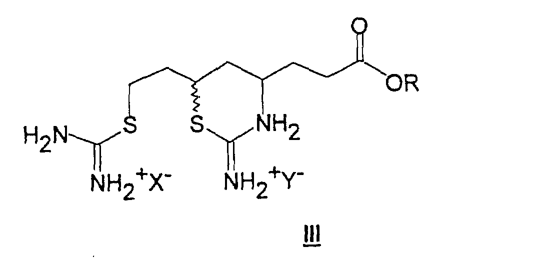 dans laquelle R représente un atome d'hydrogène ou un groupe alkyle ayant de 1 à 3 atomes de carbone, et X⁻ et Y⁻ sont identiques ou différents et représentent un anion d'un acide minéral, d'un acide alkyl- ou arylsulfonique ou d'un acide carboxylique, et de ses énantiomères, dans lequel on fait réagir avec de la thio-urée de l'acide 8-chloro-6-sulfonyloxyoctanoïque racémique ou sous forme d'énantiomère pur, ou ses esters alkyliques en C₁-C₃.

2. Procédé selon la revendication 1, dans lequel on utilise comme substituants sulfonyloxy des groupes méthylsulfonyloxy, 4-méthylphénylsulfonyloxy, perfluoroalkylsulfonyloxy.

3. Procédé selon la revendication 1 ou 2, dans lequel on isole sous forme cristalline ou huileuse, à partir du mélange réactionnel résultant, les composés de formule III selon la revendication 1, en solution aqueuse, sous forme de sels d'acides sulfoniques, d'acides minéraux ou d'acides carboxyliques.
